(19) Europäisches Patentamt
European Patent Office
Office européen des brevets

(11) Veröffentlichungsnummer: **0 025 948**
**B1**

(12) **EUROPÄISCHE PATENTSCHRIFT**

(45) Veröffentlichungstag der Patentschrift: **02.03.83**

(21) Anmeldenummer: **80105456.0**

(22) Anmeldetag: **12.09.80**

(51) Int. Cl.³: **C 07 D 233/60,** A 01 N 43/50, A 01 N 47/10, A 01 N 47/20 // C07C49/255, C07C49/16, C07C49/167, C07C143/68

(54) **Acylierte Imidazolyl-gamma-fluorpinakolyl-Derivate, Verfahren zu ihrer Herstellung und ihre Verwendung als Fungizide.**

(30) Priorität: **24.09.79 DE 2938575**

(43) Veröffentlichungstag der Anmeldung: **01.04.81 Patentblatt 81/13**

(45) Bekanntmachung des Hinweises auf die Patenterteilung: **02.03.83 Patentblatt 83/9**

(84) Benannte Vertragsstaaten: **BE CH DE FR GB IT LI NL**

(56) Entgegenhaltungen:
**EP-A-0 004 303**
**EP-A-0 006 538**
**EP-A-0 019 130**
**EP-A-2 811 916**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal 1 (DE)**
Erfinder: **Büchel, Karl Heinz, Dr., Haus an der Dachdelle Dabringhausener Strasse 42, D-5093 Burscheid (DE)**
Erfinder: **Stetter, Jörg, Dr., Gellertweg 4, D-5600 Wuppertal 1 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5623 Leichlingen (DE)**
Erfinder: **Paul, Volker, Dr., Ahornstrasse 5, D-5650 Solingen (DE)**

EP 0 025 948 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Acylierte Imidazolyl-γ-fluorpinakolyl-Derivate, Verfahren zu ihrer Herstellung und ihre Verwendung als Fungizide

Die Erfindung betrifft neue acylierte Imidazolyl-γ-fluorpinakolyl-Derivate, mehrere Verfahren zu ihrer Herstellung und ihre Verwendung als Fungizide.

Es ist bereits bekanntgeworden, daß acylierte 1-Imidazolyl-2-hydroxy-butan-Derivate, wie insbesondere im Phenylteil substituierte 2-Acyloxy- bzw. 2-Carbamoyloxy-3,3-dimethyl-1-phenoxy-1-imidazol-1-yl-butane, gute fungizide Eigenschaften aufweisen (vergleiche DE-OS 2 604 761). Deren Wirkung ist jedoch, insbesondere bei niedrigen Aufwandmengen und -konzentrationen, nicht immer ganz befriedigend.

Es wurden neue acylierte Imidazolyl-γ-fluorpinalkolyl-Derivate der allgemeinen Formel I

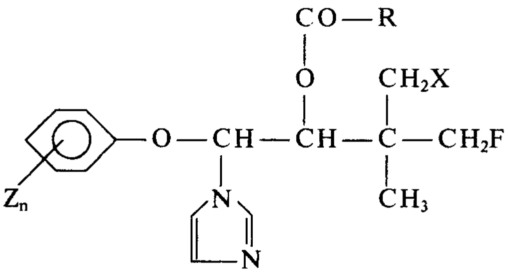

(I)

in welcher

R für Alkyl mit 1 bis 6 Kohlenstoffatomen, für 1-Methyl-vinyl, für Halogenalkyl mit 1 bis 3 Kohlenstoff- und 1 bis 5 Halogenatomen steht, für Alkoxy und Alkoxyalkyl mit 1 bis 4 Kohlenstoffatomen in jedem Alkylteil und für Cyclohexyl steht, weiterhin für Phenyl-, Phenylalkyl- und Phenoxyalkyl-Gruppen mit jeweils bis zu 2 Kohlenstoffatomen im Alkylteil steht, wobei die drei letztgenannten Gruppen im Phenylteil substituiert sein können durch Halogen, Alkyl und Alkoxy mit jeweils 1 bis 2 Kohlenstoffatomen, sodann für Alkylamino mit 1 bis 12, für Dialkylamino mit 1 bis 2 Kohlenstoffatomen in jedem Alkylteil und für Halogenalkylamino mit bis zu 4 Kohlenstoff- und bis zu 5 Halogenatomen steht, ferner für Alkoxycarbonylamino und Alkoxyalkylamino mit jeweils 1 bis 4 Kohlenstoffatomen in jedem Alkylteil steht, und schließlich noch für Phenylamino steht, welches durch Halogen substituiert sein kann,

X für Wasserstoff oder Fluor steht,

Z für Halogen, Cyano und Nitro, für Alkyl mit bis zu 4 Kohlenstoff- und für Halogenalkyl mit bis zu 2 Kohlenstoff- und bis zu 5 Halogenatomen und für Cyclohexyl steht, ferner für Alkoxycarbonyl mit insgesamt bis zu 5 und für Alkoxy und Alkylthio mit jeweils bis zu 2 Kohlenstoffatomen steht, und weiterhin für Phenyl, Phenoxy und Benzyl steht, wobei die drei letztgenannten Gruppen im Phenylteil durch Halogen substituiert sein können, und

n für ganze Zahlen von 0 bis 3 steht,

und deren physiologisch verträgliche Säureadditions-Salze und Metallsalz-Komplexe gefunden.

Die Verbindungen der allgemeinen Formel I besitzen zwei asymmetrische Kohlenstoffatome; sie können deshalb in der erythro- wie in der threo-Form vorliegen. In beiden Fällen liegen sie vorwiegend als Racemate vor.

Weiterhin wurde gefunden, daß man die acylierten Imidazolyl-γ-fluorpinakolyl-Derivate der allgemeinen Formel I erhält, wenn man 1-Imidazolyl-2-hydroxy-butan-Derivate der allgemeinen Formel II

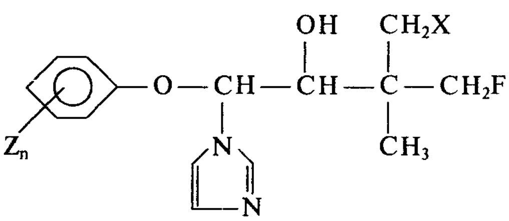

(II)

in welcher

X, Z und n die oben angegebene Bedeutung haben,

(a) mit Säurehalogeniden der allgemeinen Formel III

Hal—CO—R (III)

in welcher

R die oben angegebene Bedeutung hat und
Hal für Halogen, insbesondere Chlor oder Brom steht,

gegebenenfalls in Gegenwart eines Lösungsmittels und gegebenenfalls in Gegenwart eines Säurebindemittels umsetzt, oder

(b) mit Säureanhydriden der allgemeinen Formel IV

R—CO—O—CO—R (IV)

in welcher

R die oben angegebene Bedeutung hat,
in Gegenwart eines Lösungsmittels und gegebenenfalls in Gegenwart eines Katalysators umsetzt, oder

(c) mit Ketenen der allgemeinen Formel V

$$O{=}C{=}\underset{\displaystyle R''}{\underset{|}{C}}{-}R' \qquad (V)$$

in welcher

R′ für Wasserstoff, für Alkyl mit 1 bis 5 Kohlenstoffatomen und für Halogenmethyl mit 1 bis 3 Halogenatomen steht, und weiterhin für Halogen, für die Alkoxygruppe mit 1 bis 3 Kohlenstoffatomen und für Phenyl steht, welches gegebenenfalls durch Halogen oder Alkyl mit 1 bis 2 Kohlenstoffatomen substituiert sein kann, und

R″ die unter R′ genannten Bedeutungen annehmen kann, jedoch mit der Maßgabe, daß die Summe der Kohlenstoffatome in den Alkylresten für R′ und R″ gemeinsam um die Zahl 1 unter der Zahl derjenigen Kohlenstoffatome, die im Anspruch 1 bei der R-Definition für die Alkylreste angegeben ist, bleiben muß, und daß höchstens einer der Reste R′ und R″ für gegebenenfalls wie angegeben substituiertes Phenyl, für die Alkoxygruppe oder Halogenmethyl stehen darf,

in Gegenwart eines Lösungsmittels und gegebenenfalls in Gegenwart eines Katalysators umsetzt, oder

(d) mit Isocyanaten der allgemeinen Formel VI

O=C=N—R‴ (VI)

in welcher

R‴ für Alkyl mit 1 bis 12 Kohlenstoffatomen, für Halogenalkyl mit 1 bis zu 4 Kohlenstoff- und bis zu 5 Halogenatomen steht, sowie für Alkoxycarbonyl und Alkoxyalkyl mit jeweils 1 bis 4 Kohlenstoffatomen in jedem Alkylteil und für Phenyl steht, welches gegebenenfalls durch Halogen substituiert sein kann,

in Gegenwart eines Lösungsmittels und gegebenenfalls in Gegenwart eines Katalysators umsetzt.

Weiterhin können die erfindungsgemäß erhältlichen acylierten Imidazolyl-γ-fluorpinakolyl-Derivate der allgemeinen Formel I durch Umsetzen mit Säuren in die Salze übergeführt werden, bzw. können durch Reaktion mit Metallsalzen die entsprechenden Metallsalz-Komplexe erhalten werden.

Die neuen acylierten Imidazolyl-γ-fluorpinakolyl-Derivate weisen starke fungizide Eigenschaften auf. Dabei zeigen überraschenderweise die erfindungsgemäßen Verbindungen eine erheblich höhere Wirkung, als die aus dem Stand der Technik bekannten acylierten bzw. carbamoylierten Imidazolyl-2-hydroxy-butan-Derivate, die chemisch und wirkungsmäßig naheliegende Verbindungen sind. Die erfindungsgemäßen Stoffe stellen somit eine Bereicherung der Technik dar.

Die erfindungsgemäßen acylierten Imidazolyl-γ-fluorpinakolyl-Derivate sind durch die allgemeine Formel I definiert.

Bevorzugt sind diejenigen Verbindungen der allgemeinen Formel I, in der R für Methyl, Ethyl, Isobutyl, Chlormethyl, Dichlormethyl, Chlorethyl, Chlorpropyl, 1-Methyl-vinyl, Cyclohexyl, gegebenenfalls einfach oder mehrfach substituiertes Phenyl, Benzyl oder Phenoxymethyl mit Chlor, Brom, Methyl oder Methoxy als Substituenten, ferner für Methoxy, Ethoxy, Isopropoxy, Butoxy oder Isobutoxy, Methyl- und Ethylamino, Dimethylamino, Phenylamino, Chlorphenylamino, Chlorethylamino, Methoxycarbonylamino, Ethoxycarbonylamino und Methoxymethylamino steht, X für Wasserstoff oder Fluor steht;

Z für Chlor, Brom, Methyl, Ethyl, Cyclohexyl, Methoxy, Methylthio, Trifluormethyl, Methoxycarbonyl, Cyano, Nitro sowie gegebenenfalls durch Chlor substituiertes Phenyl, Benzyl oder Phenoxy steht und n für 0, 1 oder 2 steht.

Im einzelnen seien außer den Herstellungsbeispielen und den Beispielen der Tabelle 1 folgende Verbindungen der allgemeinen Formel I genannt:

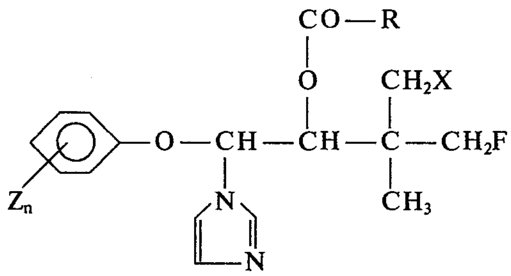

(I)

| R | X | $Z_n$ |
|---|---|---|
| $-CHCl_2$ | H | 4-Cl |
| $-CH_2Cl$ | H | 4-Cl |
| $-CH_3$ | H | 4-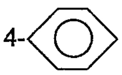 |
| —NH—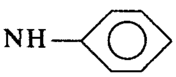 | H | 4-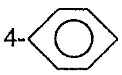 |
| —NH—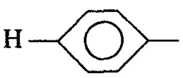—Cl | H | 4-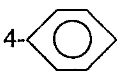 |
| $-NHCH_3$ | H | 4-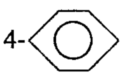 |
| $-NHCH_3$ | H | 4-Cl; 2-$CH_3$ |
| $-NHCH_3$ | H | 2-Cl |
| $-NHC_2H_5$ | H | 4-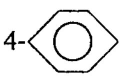 |
| $-C_4H_9$-i | H | 4-Br |
| $-CH_3$ | H | 2,4-$Cl_2$ |
| $-C_2H_5$ | H | 2,4-$Cl_2$ |
| $-CH_3$ | H | 4-$OCH_3$ |
| $-CH_2Cl$ | H | 3-$CF_3$ |
| $-CHCl_2$ | H | 4-CO—$OCH_3$ |
| $-NHCH_2OCH_3$ | H | 4-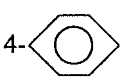 |
| $-NHCH_2OC_2H_5$ | H | 4-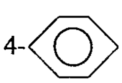 |
| —NH—$COOCH_3$ | H | 4-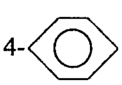 |

Fortsetzung

| R | X | $Z_n$ |
|---|---|---|
| $—NH—COOC_2H_5$ | H | 4-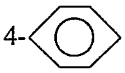 |
| $—N(CH_3)_2$ | H | 4-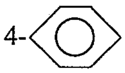 |
| $—OCH_3$ | H | 4-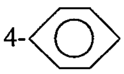 |
| $—CH_3$ | H | 4-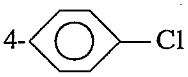 |
| $—CH_3$ | H | 4-O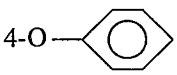 |
| $—CH_3$ | H | 4-O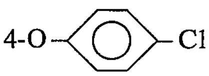 |
| $—CH_3$ | H | 4-CN |
| $—CH_3$ | H | $4\text{-}NO_2$ |
| $—CH_2OC_2H_5$ | H | $2,4\text{-}Cl_2$ |
| $—NHCH(CH_3)_2$ | H | $2,4\text{-}Cl_2$ |
| $—NH—CH_2OCH_3$ | H | $2,4\text{-}Cl_2$ |
| $—OC_2H_5$ | H | $2,4\text{-}Cl_2$ |
| $—C(CH_3){=}CH_2$ | H | $2,4\text{-}Cl_2$ |
| $—NH—CH_3$ | H | $2,4\text{-}Cl_2$ |
| $—CH_2—CH(CH_3)_2$ | H | $2,4\text{-}Cl_2$ |
| $—CH_2$—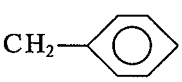 | H | $2,4\text{-}Cl_2$ |
| $—CH_2—CH_2Cl$ | H | $2,4\text{-}Cl_2$ |
| $—CH_2—CH_2—CH_2Cl$ | H | $2,4\text{-}Cl_2$ |
| NH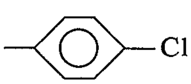 | H | $2,4\text{-}Cl_2$ |
| $—CHCl_2$ | H | $2,4\text{-}Cl_2$ |
| $—CH_2Cl$ | H | $2,4\text{-}Cl_2$ |
| 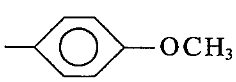 | H | $2,4\text{-}Cl_2$ |

Fortsetzung

| R | X | $Z_n$ |
|---|---|---|
| $—CH_2—O—$ 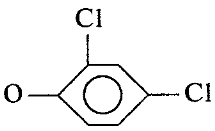 | H | 2,4-$Cl_2$ |
| 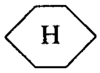 | H | 2,4-$Cl_2$ |
| 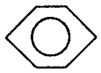 | H | 2,4-$Cl_2$ |
| 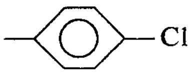 | H | 2,4-$Cl_2$ |
| $—CH_2OC_2H_5$ | H | 4-Cl |
| $—NH—CH(CH_3)_2$ | H | 4-Cl |
| $—NH—CH_2OCH_3$ | H | 4-Cl |
| $—OC_2H_5$ | H | 4-Cl |
| $—C(CH_3){=}CH_2$ | H | 4-Cl |
| $—CH_2—CH(CH_3)_2$ | H | 4-Cl |
| $—CH_2—$ 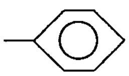 | H | 4-Cl |
| $—CH_2—CH_2Cl$ | H | 4-Cl |
| $—CH_2—CH_2—CH_2Cl$ | H | 4-Cl |
| $—NH—$ 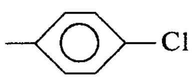 | H | 4-Cl |
| 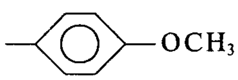 | H | 4-Cl |
| $—CH_2—O—$ 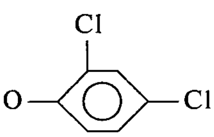 | H | 4-Cl |
| $—NH—CH_3$ | H | 4-Cl |
| $—CH_3$ | H | 4-Cl |
| 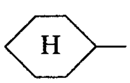 | H | 4-Cl |
| 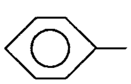 | H | 4-Cl |
| 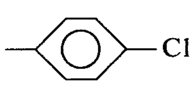 | H | 4-Cl |

Fortsetzung

| R | X | $Z_n$ |
|---|---|---|
| $—NHCH_3$ | F | 2,4-$Cl_2$ |
| $—NHCH_3$ | F | 4-Cl |
| $—CH_3$ | F | 4-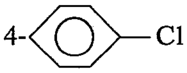 |
| $—NHCH_3$ | F | 4-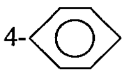 |
| $—NHC_2H_5$ | F | 4-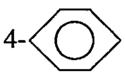 |
| $—NHCH_2OCH_3$ | F | 4-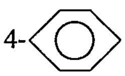 |
| $—NH—CH(CH_3)_2$ | F | 4-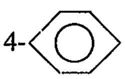 |
| $—CH_2OC_2H_5$ | F | 4-Cl |
| $—NH—CH(CH_3)_2$ | F | 4-Cl |
| $—NHCH_2OCH_3$ | F | 4-Cl |
| $—OC_2H_5$ | F | 4-Cl |
| $—C(CH_3){=}CH_2$ | F | 4-Cl |
| $—CH_2—CH(CH_3)_2$ | F | 4-Cl |
| $—CH_2$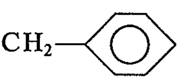 | F | 4-Cl |
| $—CH_2—CH_2Cl$ | F | 4-Cl |
| $—CH_2—CH_2—CH_2Cl$ | F | 4-Cl |
| —NH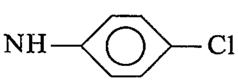 | F | 4-Cl |
| $—CHCl_2$ | F | 4-Cl |
| $—CH_2Cl$ | F | 4-Cl |
| 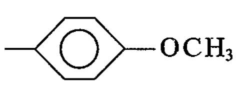 | F | 4-Cl |
| $—CH_2—O$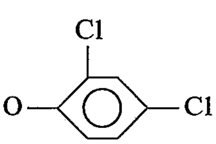 | F | 4-Cl |
| 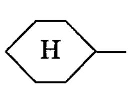 | F | 4-Cl |

Fortsetzung

| R | X | $Z_n$ |
|---|---|---|
| 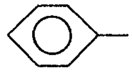 | F | 4-Cl |
| 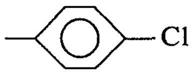 | F | 4-Cl |
| $-CH_2OC_2H_5$ | F | 2,4-$Cl_2$ |
| $-NH-CH(CH_3)_2$ | F | 2,4-$Cl_2$ |
| $-NH-CH_2OCH_3$ | F | 2,4-$Cl_2$ |
| $-OC_2H_5$ | F | 2,4-$Cl_2$ |
| $-C(CH_3){=}CH_2$ | F | 2,4-$Cl_2$ |
| $-CH_2-CH(CH_3)_2$ | F | 2,4-$Cl_2$ |
| 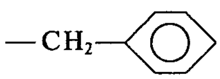 | F | 2,4-$Cl_2$ |
| $-CH_2-CH_2Cl$ | F | 2,4-$Cl_2$ |
| $-CH_2-CH_2-CH_2Cl$ | F | 2,4-$Cl_2$ |
| 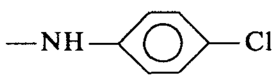 | F | 2,4-$Cl_2$ |
| $-CHCl_2$ | F | 2,4-$Cl_2$ |
| $-CH_2Cl$ | F | 2,4-$Cl_2$ |
| 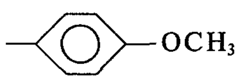 | F | 2,4-$Cl_2$ |
| 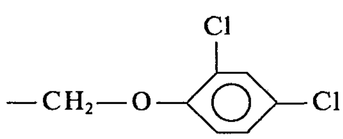 | F | 2,4-$Cl_2$ |
| 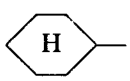 | F | 2,4-$Cl_2$ |
| 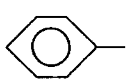 | F | 2,4-$Cl_2$ |
| 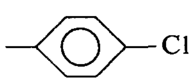 | F | 2,4-$Cl_2$ |

Verwendet man beispielsweise 1-(4-Chlorphenoxy)-1-imadazol-1-yl-3,3-dimethyl-4-fluor-butan-2-ol und Dichloracetylchlorid als Ausgangsstoffe, so kann der Reaktionsablauf durch das folgende Formelschema wiedergegeben werden (Verfahren a):

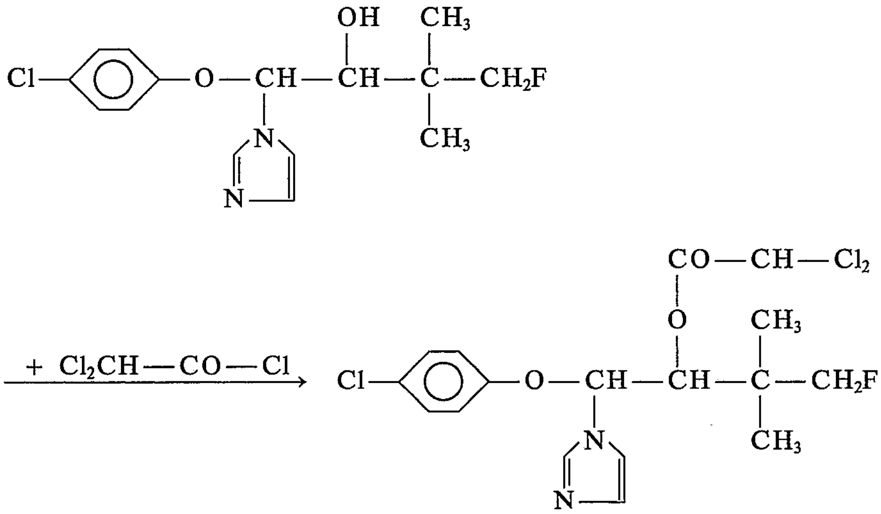


Verwendet man beispielsweise 1-(4-Chlorphenoxy)-1-imadazol-1-yl-3,3-dimethyl-4-fluor-butan-2-ol und Acetanhydrid als Ausgangsstoffe, so kann der Reaktionsablauf durch das folgende Formelschema wiedergegeben werden (Verfahren b):

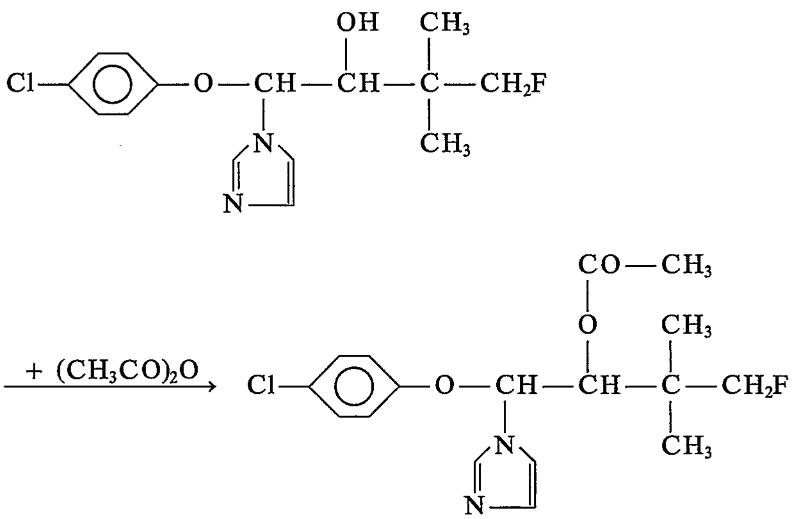


Verwendet man beispielsweise 1-(2,4-Dichlorphenoxy)-1-imadazol-1-yl-3,3-dimethyl-4-fluor-butan-2-ol und 4-Chlorphenylisocyanat als Ausgangsstoffe, so kann der Reaktionsablauf durch das folgende Formelschema wiedergegeben werden (Verfahren d):

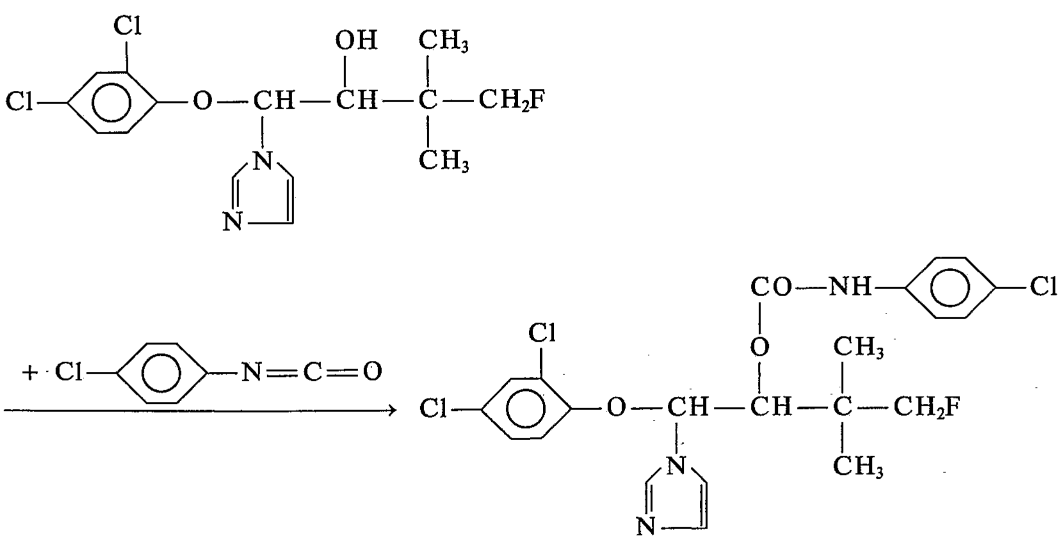

Umsetzungen von 1-Imidazolyl-2-hydroxy-butan-Derivaten der allgemeinen Formel II mit einem Keten der allgemeinen Formel V lassen sich in entsprechender Weise formulieren (Verfahren c).

Die für alle Verfahrensvarianten als Ausgangsstoffe zu verwendenden 1-Imidazolyl-2-hydroxy-butan-Derivate sind durch die allgemeine Formel II definiert. In dieser Formel stehen X, Z und der Index n vorzugsweise für diejenigen Reste, die bereits im Zusammenhang mit der Beschreibung der erfindungsgemäßen Stoffe der allgemeinen Formel I vorzugsweise für diese Substituenten genannt wurden.

Die 1-Imidazolyl-2-hydroxy-butan-Derivate der allgemeinen Formel II sind noch nicht bekannt; sie sind jedoch Gegenstand einer eigenen älteren Anmeldung (vergleiche DE-OS 2 918 893) und können hergestellt werden, indem man Halogenetherketone der allgemeinen Formel VII

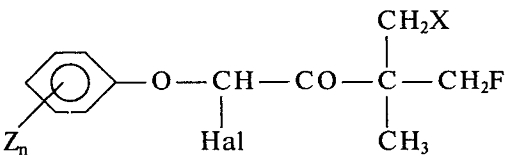

(VII)

in welcher

X, Z und n die oben angegebene Bedeutung haben und
Hal für Halogen, vorzugsweise Chlor oder Brom steht,

mit Imidazol in Gegenwart eines Säurebindemittels, wie beispielsweise Natriumcarbonat oder ein Überschuß an Imidazol und gegebenenfalls in Gegenwart eines inerten organischen Lösungsmittels, wie beispielsweise Aceton oder Acetonitril, bei Temperaturen zwischen 60 und 120°C umsetzt und die erhaltenen Keto-Derivate nach bekannten Methoden reduziert, wie z. B. durch Umsetzung mit komplexen Hydriden, wie insbesondere Natriumborhydrid, gegebenenfalls in Gegenwart eines polaren organischen Lösungsmittels, wie beispielsweise Aolkohole, bei Temperaturen zwischen 0 und 30°C; oder durch Umsetzung mit Aluminiumisopropylat in Gegenwart eines inerten organischen Lösungsmittels, wie beispielsweise Isopropanol, bei Temperaturen zwischen 20 und 120°C. Die Aufarbeitung erfolgt in üblicher Weise.

Die Halogenetherketone der allgemeinen Formel VII sind noch nicht bekannt. Sie sind jedoch ebenfalls Gegenstand der oben genannten eigenen älteren Anmeldung und können nach bekannten Verfahren (vergleiche z. B. DE-OS 2 632 602) erhalten werden, indem man z. B. bekannte Phenole der allgemeinen Formel VIII

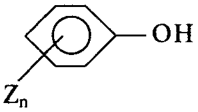

(VIII)

in welcher

Z und n die oben angegebene Bedeutung haben,

mit einem Halogenketon der allgemeinen Fohrmel IX

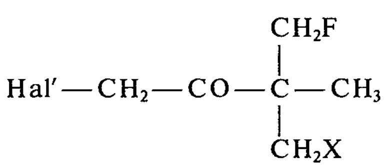

(IX)

in welcher

X die oben angegebene Bedeutung hat und
Hal' für Chlor oder Brom steht,

umsetzt. Das noch verbliebene aktive Wasserstoffatom wird anschließend in üblicher Weise gegen Halogen ausgetauscht (vgl. auch die Herstellungsbeispiele).

Die Halogenketone der allgemeinen Formel IX sind ebenfalls Gegenstand der oben genannten eigenen älteren Anmeldung. Sie können auf allgemein übliche und bekannte Weise erhalten werden, indem man Fluor-derivate des 3,3-Dimethyl-butan-2-ons der allgemeinen Formel X

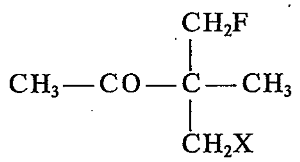

(X)

in welcher

X die oben angegebene Bedeutung hat,

mit Chlor und Brom in Gegenwart eines inerten organischen Lösungsmittels, wie beispielsweise Ether oder chlorierte Kohlenwasserstoffe, bei Raumtemperatur versetzt (vergleiche auch die Herstellungsbeispiele), oder mit üblichen Chlorierungsmitteln, wie beispielsweise Sulfurylchlorid bei 20 bis 60°C umsetzt.

Die Fluorderivate des 3,3-Dimethyl-butan-2-ons der allgemeinen Formel X sind Gegenstand einer eigenen älteren Patentanmeldung (vergleiche DE-OS 2 843 767). Man erhält die Fluorderivate des 3,3-Dimethyl-butan-2-ons der allgemeinen Formel X, wenn man Sulfonsäureester der allgemeinen Formel XI

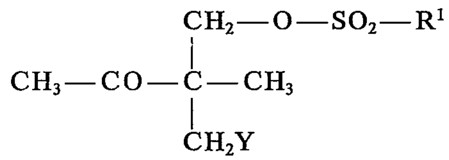

(XI)

in welcher

$R^1$ für Alkyl mit 1 bis 4 Kohlenstoffatomen, insbesondere Methyl, oder Aryl mit 6 bis 12 Kohlenstoffatomen, insbesondere Phenyl oder Tolyl, steht und

Y für Wasserstoff oder die Gruppe $-O-SO_2-R^1$ steht,

mit Metallfluoriden, wie beispielsweise Natrium- und Kaliumfluorid, in Gegenwart eines polaren organischen Lösungsmittels, wie beispielsweise Di-, Tri- oder Tetraethylenglykol, bei Temperaturen zwischen 80 und 250°C umsetzt (vgl. auch die Herstellungsbeispiele).

Die Sulfonsäureester der allgemeinen Formel XI sind teilweise bekannt [J. Org. Chem. 35, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (siehe z. B. Houben—Weyl, Methoden der Org. Chemie, Bd. IX, S. 388 und 663, sowie die Angaben bei den Herstellungsbeispielen).

Im einzelnen seien als Beispiele für die Ausgangsstoffe der allgemeinen Formel II genannt:

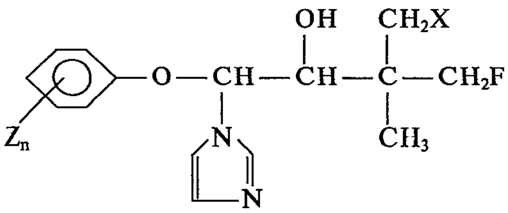

(II)

| Zn | X | Zn | X |
|---|---|---|---|
| – | H | – | F |
| 2-Cl | H | 2-Cl | F |
| 3-Cl | H | 3-Cl | F |
| 4-Cl | H | 4-Cl | F |
| 2-F | H | 2-F | F |
| 3-F | H | 3-F | F |
| 4-F | H | 4-F | F |
| 2-Br | H | 2-Br | F |
| 3-Br | H | 3-Br | F |
| 4-Br | H | 4-Br | F |
| 2,4-$Cl_2$ | H | 2,4-$Cl_2$ | F |
| 2-$CH_3$ | H | 2-$CH_3$ | F |
| 4-$CH_3$ | H | 4-$CH_3$ | F |
| 2-Cl, 4-$CH_3$ | H | 2-Cl, 4-$CH_3$ | F |
| 4-Cl, 2-$CH_3$ | H | 4-Cl, 2-$CH_3$ | F |
| 4-J | H | 4-J | F |
| 4-CN | H | 4-CN | F |
| 2-$NO_2$ | H | 2-$NO_2$ | F |
| 4-$COOCH_3$ | H | 4-$COOCH_3$ | F |
| 4-$COOC_2H_5$ | H | 4-$COOC_2H_5$ | F |
| 4-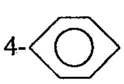 | H | 4-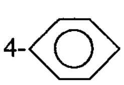 | F |
| 2-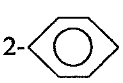 | H | 2-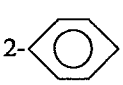 | F |
| 4-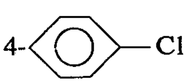 | H | 4-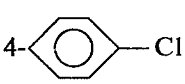 | F |

Die außerdem für die Verfahrensvariante (a) als Ausgangsstoffe zu verwendenden Säurehalogenide sind durch die allgemeine Formel III definiert. In dieser Formel steht R vorzugsweise für diejenigen Reste, die bereits im Zusammenhang mit der Beschreibung der erfindungsgemäßen Stoffe der allgemeinen Formel I vorzugsweise für diesen Substituenten genannt wurden.

Die Säurehalogenide der allgemeinen Formel III sind bekannt oder lassen sich nach üblichen Verfahren herstellen; so z. B. durch Umsetzung von Carbonsäuren bzw. deren Alkalisalzen mit Säurehalogeniden des Phosphors oder Schwefels. Diese Methoden sind aus den allgemeinen Lehrbüchern der organischen Chemie bekannt.

Die weiterhin für die Verfahrensvariante (b) als Ausgangsstoffe zu verwendenden Säureanhydride sind durch die allgemeine Formel IV definiert. In dieser Formel steht R vorzugsweise für diejenigen Reste, die bereits im Zusammenhang mit der Beschreibung der erfindungsgemäßen Stoffe der allgemeinen Formel I vorzugsweise für diesen Substituenten genannt wurden.

Die Säureanhydride der allgemeinen Formel IV sind bekannt oder lassen sich nach bekannten Verfahren herstellen; so z. B. durch Einwirkung von Säurechloriden auf die Alkalisalze der Carbonsäuren.

Die außerdem für die Verfahrensvariante (c) als Ausgangsstoffe zu verwendenden Ketene sind durch die allgemeine Formel V definiert.

Die Ketene der allgemeinen Formel V sind bekannte oder lassen sich nach bekannten Verfahren herstellen, so z. B. durch Thermolyse von Ketonen oder durch Dehydratisierung von Carbonsäuren (vgl. Houben–Weyl, »Methoden der organischen Chemie«, Band 7/4, Georg Thieme Verlag).

Die weiterhin für die Verfahrensvariante (d) als Ausgangsstoffe zu verwendenden Isocyanate sind durch die allgemeine Formel VI definiert.

Die Isocyanate der allgemeinen Formel VI sind bekannt oder lassen sich nach allgemein üblichen und bekannten Verfahren herstellen; so z. B. durch Umsetzen von Aminen mit Phosgen und anschließendem Erhitzen.

Als Lösungsmittel kommen für die Umsetzung gemäß Verfahrensvariante (a) vorzugsweise alle inerten organischen Lösungsmittel in Frage. Hierzu gehören vorzugsweise Nitrile, wie Propionitril, insbesondere Acetonitril; Ether, wie Tetrahydrofuran oder Dioxan; Ester, wie Essigsäureethylester; aromatische Kohlenwasserstoffe, wie Benzol oder Toluol und halogenierte Kohlenwasserstoffe, wie Methylenchlorid, Tetrachlorkohlenstoff oder Chloroform. Der Einfachheit halber kann das eingesetzte Säurechlorid auch als Lösungsmittel verwendet werden, womit ein entsprechender Überschuß notwendig wird.

Die Reaktionstempreraturen können bei der Durchführung der Verfahrensvariante (a) in einem größeren Bereich variiert werden. Im allgemeinen arbeitet man zwischen 0 und 100°C, vorzugsweise zwischen 20 und 85°C. Bei Anwesenheit eines Lösungsmittels wird gegebenenfalls beim Siedepunkt des jeweiligen Lösungsmittels gearbeitet.

Das erfindungsgemäße Verfahren (a) kann gegebenenfalls in Gegenwart von Säurebindern (Halogenwasserstoff-Akzeptoren) durchgeführt werden; als solche können alle üblichen Säurebindungsmittel verwendet werden. Hierzu gehören organische Basen, vorzugsweise tertiäre Amine, wie z. B. Triäthylamin; ferner anorganische Basen, wie z. B. Alkalihydroxide und Alkalicarbonate.

Bei der Durchführung der Verfahrensvariante (a) arbeitet man vorzugsweise in molaren Mengen. Die Verbindungen der allgemeinen Formel I fallen in Form ihrer Hydrohalogenide an und können als solche isoliert werden, indem man sie durch Zugabe eines organischen Solvents, z. B. Hexan ausfällt, absaugt und gegebenenfalls durch Umkristallisation reinigt. Die Verbindungen können auch in Form ihrer freien Base isoliert werden, indem man das Reaktionsgemisch mit wäßriger Natriumhydrogencarbonatlösung versetzt und die Base nach üblichen Methoden isoliert.

Als Verdünnungsmittel kommen für die Umsetzung gemäß Verfahrensvariante (b) vorzugsweise alle inerten organischen Lösungsmittel infrage. Hierzu gehören vorzugsweise die bei der Verfahrensvariante (a) aufgezählten Solventien sowie die jeweils verwendeten Säureanhydride der allgemeinen Formel IV.

Als Katalysatoren können bei der Verfahrensvariante (b) vorzugsweise alle üblichen sauren und basischen Katalysatoren verwendet werden, wie z. B. Schwefelsäure, Chlorwasserstoff, Bromwasserstoff, Bortrifluorid, Zinkchlorid, Natriumacetat, Natriumbenzoat, Natriumcarbonat, Calciumoxid, Magnesiumoxid.

Die Reaktionstemperaturen können bei der Durchführung der Verfahrensvariante (b) in einem größeren Bereich variiert werden. Im allgemeinen arbeitet man zwischen 0 und 150°C, vorzugsweise zwischen 80 und 120°C.

Bei der Durchführung der Verfahrensvariante (b) arbeitet man vorzugsweise in molaren Mengen. Der Einfachheit halber kann man das eingesetzte Säureanhydrid der allgemeinen Formel IV auch als Lösungsmittel verwenden, womit ein entsprechender Überschuß erforderlich wird. Die Isolierung der Verbindungen der allgemeinen Formel I erfolgt in üblicher Weise.

Als Verdünnungsmittel kommen für die Umsetzung gemäß Verfahrensvariante (c) vorzugsweise alle inerten organischen Lösungsmittel infrage. Hierzu gehören vorzugsweise die bei der Verfahrensvariante (a) aufgeführten Solventien.

Als Katalysatoren können bei der Verfahrensvariante (c) vorzugsweise alle üblichen sauren und basischen Katalysatoren verwendet werden. Hierzu gehören vorzugsweise die bei der Verfahrensvariante (b) aufgezählten Stoffe.

Die Reaktionstemperaturen können bei der Durchführung der Verfahrensvariante (c) in einem bestimmten Bereich variiert werden. Im allgemeinen arbeitet man zwischen —10 und 70°C, vorzugsweise zwischen 0 und 40°C.

Bei der Durchführung der Verfahrensvariante (c) arbeitet man vorzugsweise in molaren Mengen. Die Isolierung der Verbindungen der allgemeinen Formel I erfolgt nach üblichen Methoden.

Als Verdünnungsmittel kommen für die Umsetzung gemäß Verfahrensvariante (d) vorzugsweise alle inerten organischen Lösungsmittel infrage. Hierzu gehören vorzugsweise die bei der Verfahrensvariante (a) aufgezählten Solventien.

Als Katalysatoren können beim Verfahren (d) vorzugsweise verwendet werden: tertiäre Basen, wie Triethylamin und Pyridin oder Zinn-organische Verbindungen, wie Dibutylzinndilaurat.

Die reaktionstemperaturen können bei der Durchführung der Verfahrensvariante (d) in einem größeren Bereich variiert werden. Im allgemeinen arbeitet man zwischen 0 und 100°C, vorzugsweise zwischen 20 und 40°C.

Bei der Durchführung der Verfahrensvariante (d) arbeitet man vorzugsweise in molaren Mengen. Zur Isolierung der Verbindungen der allgemeinen Formel I wird das Lösungsmittel abdestilliert und der Rückstand nach üblichen Methoden aufgearbeitet.

Zur Herstellung von physiologisch verträglichen Säureadditionssalzen der Verbindungen der allgemeinen Formel I kommen vorzugsweise folgende Säuren infrage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure, sowie Sulfonsäure, wie z. B. p-Toluolsulfonsäure und 1,5-Naphthalindisulfonsäure. Die Säureadditions-Salze können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen kommen vorzugsweise Salze von Metallen der II. bis IV. Haupt- und der I. und II. sowie IV. bis VIII. Nebengruppe infrage, wobei Kupfer, Zink, Mangen, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe können in einfacher Weise nach üblichen Verfahren erhalten werden, so z. B. durch Lösen des Metallsalzes in Alkohol, z. B. Ethanol und Hinzufügen zur Verbindung der allgemeinen Formel I. Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bedämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bedämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen; so zur Bekämpfung von Podosphaera-Arten, wie z. B. gegen den Erreger des Apfelmehltaus (Podosphaera leucotricha), von Erysiphe-Arten, wie z. B. gegen den Erreger des Gurkenmehltaus (Erysiphe cichoracearum) oder des Getreidemehltaus (Erysiphe graminis); sowie zur Bekämpfung anderer Getreidekrankheiten, wie Getreiderost.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan

oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenxoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

In bestimmten Aufwandmengen zeigen die erfindungsgemäßen Stoffe auch eine wachstumsregulierende Wirkung.

Herstellungsbeispiele

Beispiel 1

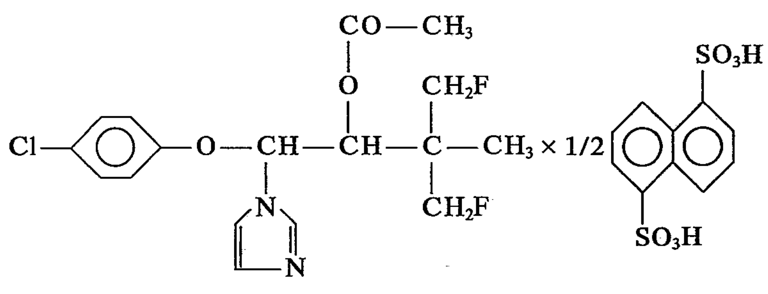

(Verfahren b)

12,2 g (0,037 Mol) 3,3-Bisfluormethyl-1-(4-chlorphenoxy)-1-(imidazol-1-yl)-butan-2-ol werden in 70 ml Acetanhydrid gelöst. Man läßt 16 Stunden bei 100°C rühren, destilliert das überschüssige Acetanhydrid im Vakuum ab und nimmt den Rückstand in 300 ml Methylenchlorid auf. Die organische Phase wird zweimal mit je 800 ml Wasser gewaschen, über Natriumsulfat getrocknet und eingeengt. Der Rückstand wird mit 100 ml Aceton aufgenommen und mit einer Lösung von 9 g (0,038 Mol)

1,5-Naphthalindisulfonsäure in 50 ml Aceton versetzt. Nach 2 Stunden wird der entstandene Niederschlag abgesaugt und getrocknet. Man erhält 13 g (68%der Theorie) 2-Acetoxy-3,3-bisfluormethyl-1-(4-chlorphenoxy)-1-(imidazol-1-yl)-butan-naphthalindisulfonat-(1,5) vom Schmelzpunkt 221–224°C.

Herstellung der Vorstufen

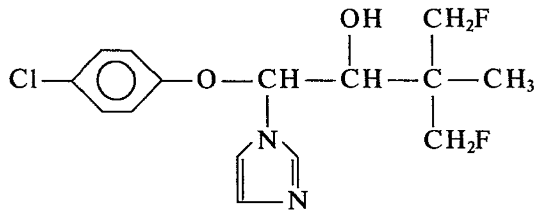

31,9 g (0,097 Mol) 3,3-Bisfluormethyl-1-(4-chlorphenoxy)-1-(imidazol-1-yl)-butan-2-on werden in 600 ml Methanol gelöst und bei 0 bis 5°C portionsweise mit 5,45 g (0,14 Mol) Natriumborhydrid versetzt. Man läßt 15 Stunden nachrühren, tropft 15 ml konzentrierte Salzsäure zu und verrührt 2 Stunden bei Raumtemperatur. Danach wird das Reaktionsgemisch in 800 ml wäßrige, gesättigte Natriumhydrogencarbonat-Lösung eingerührt. Man extrahiert mit 1000 ml Methylenchlorid, wäscht die organische Phase zweimal mit je 1000 ml Wasser, trocknet über Natriumsulfat und engt durch Abdestillieren des Lösungsmittels im Vakuum ein. Das zurückbleibende Öl wird mit Isopropylether verrührt. Man erhält 22,2 g (67,5% der Theorie) 3,3-Bisfluormethyl-1-(4-chlorphenoxy)-1-(imidazol-1-yl)-butan-2-ol vom Schmelzpunkt 137°C.

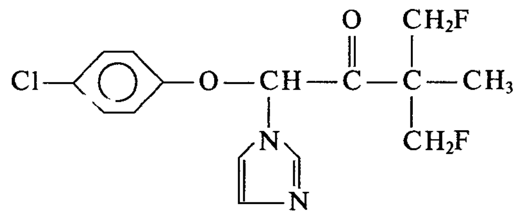

61,5 g (0,18 Mol) 3,3-Bisfluormethyl-1-brom-(4-chlorphenoxy)-butan-2-on werden mit 27,2 g (0,4 Mol) Imidazol in 500 ml Acetonitril 4 Stunden bei 45°C gerührt. Das Lösungsmittel wird im Wasserstrahlvakuum abdestilliert, das zurückbleibende Öl in 500 ml Methylenchlorid aufgenommen, die organische Phase zweimal mit 1000 ml Wasser gewaschen, über Natriumsulfat getrocknet und das Lösungsmittel abdestilliert. Das Öl wird in Aceton aufgenommen, mit 36 g (0,1 Mol) 1,5-Naphthalindisulfonsäure-tetrahydrat versetzt und der entstehende Niederschlag abgesaugt. Der Niederschlag wird mit Natriumhydrogencarbonatlösung behandelt.

Man erhält 14 g (24% der Theorie) 3,3-Bisfluormethyl-1-(4-chlorphenoxy)-1-(imidazol-1-yl)-butan-2-on als zähflüssiges Öl, das direkt weiter umgesetzt wird.

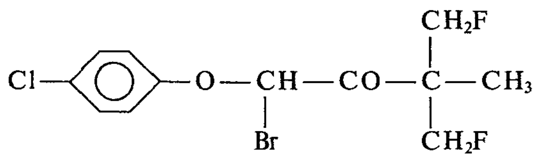

166 g (0,632 Mol) 3,3-Bisfluormethyl-1-(4-chlorphenoxy)-butan-2-on werden in 500 ml Methylenchlorid gelöst und bei 20 bis 30°C unter Rühren und Kühlen tropfenweise mit 100 g (0,625 Mol) Brom versetzt. Es wird 2 Stunden bei 20°C nachgerührt. Nach Abdestillieren des Lösungsmittels im Vakuum wird der Rückstand aus Petrolether kristallisiert. Man erhält 190 g (88% der Theorie) 3,3-Bisfluormethyl-1-brom-1-(4-chlorphenoxy)-butan-2-on vom Schmelzpunkt 54–55°C.

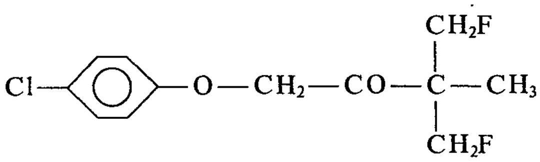

Zu einer gerührten Mischung aus 102 g (0,79 Mol) p-Chlorphenol und 110 g (0,79 Mol) gepulvertem Kaliumcarbonat in 500 ml Aceton werden bei 20 bis 30°C 171,2 g (0,79 Mol) 3,3-Bisfluormethyl-1-brom-butan-2-on zugetropft. Es wird 4 Stunden bei 40°C nachgerührt, das anorganische Salz abfiltriert und das Filtrat eingeengt. Der Rückstand wird im Hochvakuum destilliert. Man erhält 190,5 g (90% der Theorie) 3,3-Bisfluormethyl-1-(4-chlorphenoxy)-butan-2-on vom Siedepunkt 113–117°C/ 0,1 mm Hg-Säule.

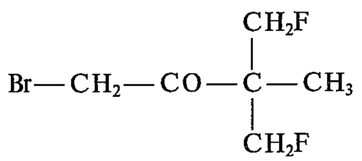

In eine Mischung aus 757 g (5,58 Mol) 3,3-Bisfluormethyl-2-butanon und 4,5 l Methylenchlorid werden bei 20 bis 30°C unter Kühlen und Rühren 903 g Brom langsam eingetropft. Die gelbliche Lösung wird noch 1 Stunde bei 20°C nachgerührt. Nach Abdestillieren des Lösungsmittels wird der Rückstand im Vakuum destilliert. Man erhält 1030 g (86% der Theorie) 3,3-Bisfluormethyl-1-brom-butan-2-on vom Siedepunkt 49–53°C/0,15 mm Hg-Säule.

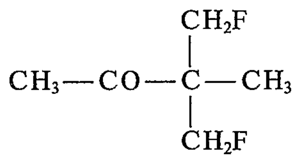

In einem Dreihalskolben mit Rührer, Tropftrichter und Liebigkühler mit gekühlter Vorlage werden 400 ml Tetraethylenglykol und 46,4 g Kaliumfluorid (0,8 Mol) vorgelegt und auf 170°C aufgeheizt. Man legt an den Vorstoß des Liebigkühlers ein Wasserstrahlvakuum (Druck ca. 20 bis 30 mbar) an. Dann werden während 45 Minuten 57,6 g (0,2 Mol) 2-Acetyl-2-methyl-propan-1,3-diol-bismethansulfat, gelöst in 100 ml Tetraethylenglykol, zugetropft. Das entstehende 3,3-Bisfluormethyl-butan-2-on wird während der Reaktion in die gekühlte Vorlage abdestilliert. Nach dem Zutropfen wird noch während 1 Stunde bei 175°C weiter destilliert.

Das aufgefangene Destillat wird anschließend redestilliert. Man erhält 14 g (ca. 51,5% der Theorie) 3,3-Bisfluormethyl-butan-2-on vom Siedepunkt 43–46°C/12 mm Hg-Säule.

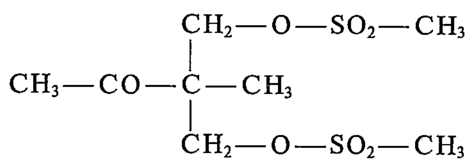

66 g (0,5 Mol) 3-Oxa-2,2-bis-(hydroxymethyl)-butan (zur Herstellung vgl. Beilstein H 1, E III 3306, IV 4132 und J. Chem. Soc., London, 1932, 2671) werden in 300 ml 1,2-Dichlorethan gelöst, 114,5 g (1 Mol) Methansulfonsäurechlorid zugetropft und bei 0–5°C 158 g (2 Mol) Pyridin zugetropft. Man läßt 15 Stunden bei Raumtemperatur nachrühren und gibt dann den Ansatz auf 600 ml Eiswasser und 100 ml konzentrierter Salzsäure. Dabei fällt ein Feststoff aus, der abgesaugt wird. Die wäßrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Ether suspendiert. Der Rückstand wird abgesaugt und mit 100 ml Ether gewaschen. Man erhält 100 g (ca 70% der Theorie) 2-Acetyl-2-methyl-propan-1,3-diol-bismethansulfonat vom Schmelzpunkt 105–108°C.

Beispiel 2

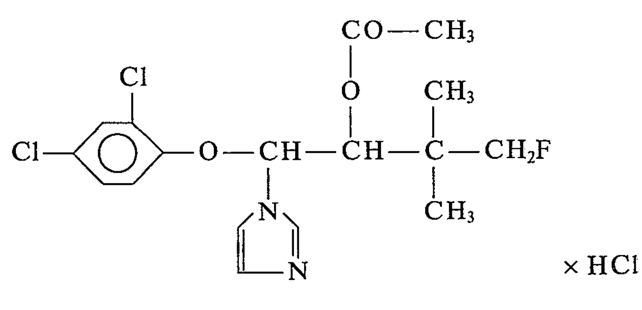

(Verfahren b)

17,3 g (0,05 Mol) 1-(2,4-Dichlorphenoxy)-3,3-dimethyl-4-fluor-1-(imidazol-1-yl)-butan-2-ol werden in 100 ml Essigsäureanhydrid gelöst und nach Zugabe von 0,1 g Natriumacetat 10 Stunden bei 100°C gerührt. Danach läßt man die Reaktionslösung auf Raumtemperatur abkühlen, rührt sie in 500 ml Wasser ein und läßt 15 Stunden stehen. Die wäßrige Phase wird zweimal mit je 200 ml Methylenchlorid extrahiert. Die vereinigten organischen Phasen werden mit je 100 ml Natriumhydrogencarbonat-Lösung und Wasser gewaschen, über Natriumsulfat getrocknet und im Vakuum eingeengt. Der Rückstand wird in 100 ml Ether aufgenommen, mit 20 ml etherischer Salzsäure versetzt, das Lösungsmittel im Vakuum abdestilliert und der Rückstand mit 50 ml Ether verrührt. Man erhält 15,3 g (72% der Theorie) 2-Acetoxy-1-(2,4-dichlorphenoxy)-3,3-dimethyl-4-fluor-1-(imidazol-1-yl)-butan-hydrochlorid vom Schmelzpunkt 198–200°C.

Herstellung der Vorstufen

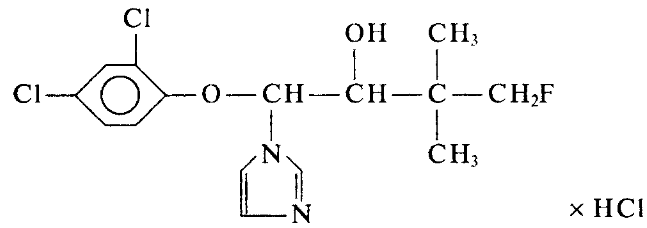

44 g (0,1275 Mol) 1-(2,4-Dichlorphenoxy)-3,3-dimethyl-4-fluor-1-(imidazol-1-yl)-2-butanon werden in 300 ml Methanol gelöst und bei 0 bis 5°C mit 6,3 g Natriumborhydrid versetzt. Man läßt 15 Stunden bei Raumtemperatur nachrühren, tropft 60 ml konzentrierte Salzsäure unter Eiskühlung zu, verrührt 10 Stunden bei Raumtemperatur und destilliert das Lösungsmittel im Wasserstrahlvakuum ab. Der Rückstand wird in 250 ml Methylenchlorid aufgenommen, in 500 ml wäßrige, gesättigte Natriumhydrogencarbonatlösung eingerührt, die Methylenchloridphase abgetrennt, dreimal mit je 100 ml Wasser gewaschen, die organische Phase über Natriumsulfat getrocknet und das Lösungsmittel abdestilliert. Das zurückbleibende Öl wird in 200 ml Ether aufgenommen, 50 ml etherische Salzsäure zugegeben und das Lösungsmittel abdestilliert. Man erhält 28,2 g (58% der Theorie) 1-(2,4-Dichlorphenoxy)-3,3-dimethyl-4-fluor-1-(imidazol-1-yl)-2-butanol-hydrochlorid vom Schmelzpunkt 184–210°C.

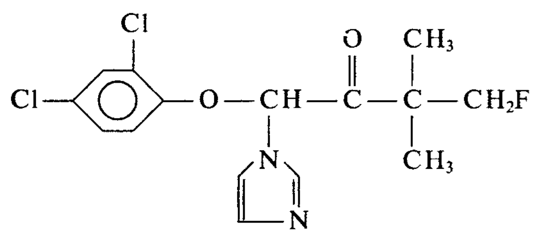

157,5 g (0,44 Mol) 1-Brom-1-(2,4-dichlorphenoxy)-3,3-dimethyl-4-fluor-2-butanon werden in 500 ml Acetonitril gelöst und zu einer Lösung von 109 g (1,6 Mol) Imidazol in 600 ml Acetonitril getropft. Man erhitzt 10 Stunden unter Rückfluß. Danach wird das Lösungsmittel im Wasserstrahlvakuum abdestilliert, der Rückstand in 1000 ml Methylenchlorid aufgenommen und dreimal mit je 500 ml Wasser gewaschen. Die organische Phase wird über Natriumsulfat getrocknet, filtriert und im Wasserstrahlvakuum durch Abdestillieren des Lösungsmittels eingeengt. Der Rückstand wird in 500 ml Ethanol gelöst, mit je 40 ml konzentrierter Salzsäure versetzt und das Lösungsmittel im Wasserstrahlvakuum abdestilliert. Der Rückstand wird mit 500 ml Ether verrührt, wobei es zur Kristallisation kommt. Man erhält 66 g (39,3% der Theorie) 1-(2,4-Dichlorphenoxy)-3,3-dimethyl-4-fluor-1-(imidazol-1-yl)-2-butanon-hydrochlorid vom Schmelzpunkt 91–110°C.

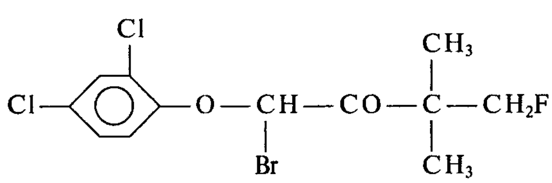

199,5 g (0,71 Mol) 1-(2,4-Dichlorphenoxy)-3,3-dimethyl-4-fluor-2-butanon werden in 500 ml Chloroform gelöst und bei 20 bis 30°C unter Rühren und Kühlen tropfenweise mit 114 g (0,71 Mol) Brom versetzt. Es wird 2 Stunden bei 20°C nachgerührt, vorsichtig mit 200 ml Wasser versetzt, die Chloroformphase mehrmals mit Eiswasser gewaschen und über Natriumsulfat getrocknet. Nach Abdestillieren des Lösungsmittels im Vakuum erhält man 205,2 g (78% der Theorie) 1-Brom-1-(2,4-dichlorphenoxy)-3,3-dimethyl-4-fluor-2-butanon als Öl, das direkt weiter umgesetzt wird.

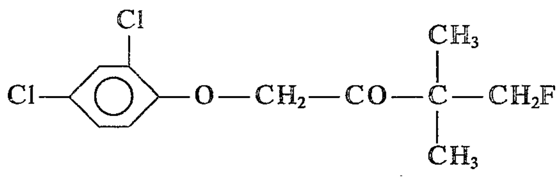

Zu einer gerührten Mischung aus 129 g (0,79 Mol) 2,4-Dichlorphenol und 110 g (0,79 Mol) gepulvertem Kaliumcarbonat in 500 ml Aceton werden unter Kühlen bei 20 bis 30°C 157 g (0,79 Mol) 1-Brom-3,3-dimethyl-4-fluor-2-butanon zugetropft. Es wird 2 Stunden bei 20°C nachgerührt, das anorganische Salz abfiltriert und das Filtrat eingeengt. Man erhält 199,3 g (90% der Theorie) 1-(2,4-Dichlorphenoxy)-3,3-dimethyl-4-fluor-2-butanon als Öl, das direkt weiter umgesetzt wird.

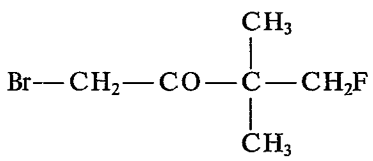

In eine Mischung aus 354 g (3 Mol) 3,3-Dimethyl-4-fluor-2-butanon und 2000 ml Ether werden bei 20 bis 30°C unter Kühlen und Rühren 480 g Brom langsam eingetropft. Die gelbliche Lösung wird noch 1 Stunde bei 20°C nachgerührt und anschließend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80–90°C/11 mm Hg-Säule.

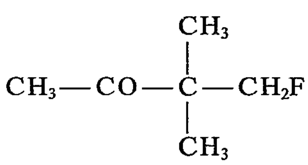

Zu der in einem Dreihals-Rührkolben mit absteigendem Kühler befindlichen Suspension von 23,2 g (0,4 Mol) trockenem Kaliumfluorid in 400 ml destilliertem Tetraethylenglykol werden bei 160°C und 20 mbar 38,8 g (0,2 Mol) 2,2-Dimethyl-2-oxobutyl-methansulfonat im Verlauf von 2 Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon, vom Siedepunkt 130–134°C.

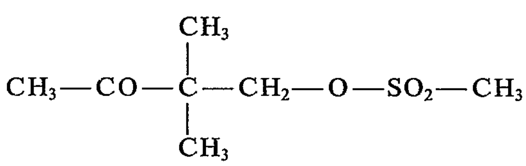

232 g (2 Mol) 3,3-Dimethyl-4-hydroxy-2-butanon (z. Herstellung vgl. Beilstein H 1 E III 3239, IV 4030 und Bull. Soc. Chim. France 1964, 2849) werden in 700 ml absolutem Pyridin bei 0 bis 5°C mit 229 g (2 Mol) Methansulfochlorid umgesetzt. Nach 12 Stunden Stehen bei 20°C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxo-butyl-methansulfonat vom Siedepunkt 106–120°C/0,12 mm Hg-Säule.

In entsprechender Weise und gemäß den Verfahren (a) bis (d) werden die nachfolgenden Beispiele der allgemeinen Formel I

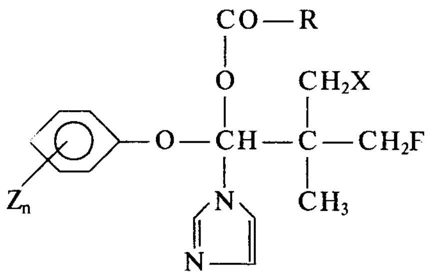

(I)

erhalten:

| Bsp. Nr. | $Z_n$ | R | X | Schmelzpunkt (°C) |
|---|---|---|---|---|
| 3 | 4-F | $-CH_3$ | F | 200–210 (× ½ NDS) |
| 4 | 2,4-$Cl_2$ | $-CH_3$ | F | 195–205 (× ½ NDS) |
| 5 | 4-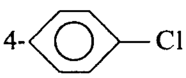 | $-CH_3$ | F | 256 (× ½ NDS) |
| 6 | 2,4-$Cl_2$ | 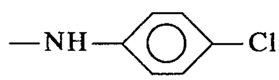 | F | 142–44 |
| 7 | 4-Cl | 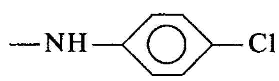 | F | 146–48 |
| 8 | 4-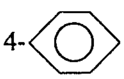 | 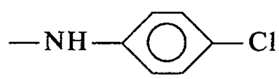 | F | 124 |
| 9 | 2,4-$Cl_2$ | 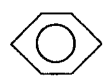 | F | 140–42 |
| 10 | 4-Cl | 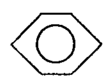 | F | 58–60 |
| 11 | 4-Cl | $-CH_2CH_2CH_2Cl$ | F | 225–28 (× ½ NDS) |
| 12 | 4-Cl | 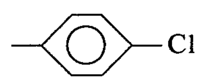 | F | > 260 (× ½ NDS) |
| 13 | 2,4-Cl | 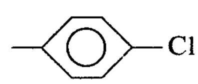 | F | > 260 (× ½ NDS) |
| 14 | 2,4-$Cl_2$ | $-CH_2CH_2CH_2Cl$ | F | 255–60 (× ½ NDS) |
| 15 | 2,4-Cl | $CHCl_2$ | F | 215–225 (× ½ NDS) |
| 16 | 2,4-$Cl_2$ | $NHCH_3$ | F | 250 (× ½ NDS) |
| 17 | 4-Cl | $NHCH_3$ | F | 121–27 |
| 18 | 4-Cl | $CH_2-OC_2H_5$ | F | 225–27 (× ½ NDS) |
| 19 | 2,4-Cl | $CH_2OC_2H_5$ | F | 222–4 (× ½ NDS) |

## Anwendungsbeispiele

In den nachfolgenden Beispielen werden die nachstehend angegebenen bekannten Verbindungen als Vergleichssubstanzen eingesetzt:

(A) 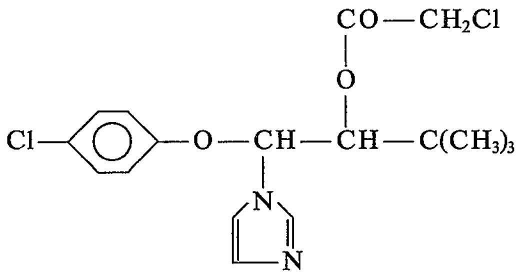 × HCl

(B) 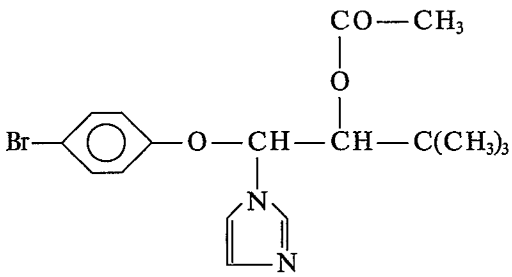

(C) 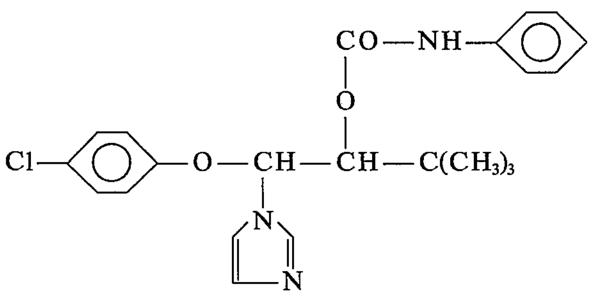

(D) 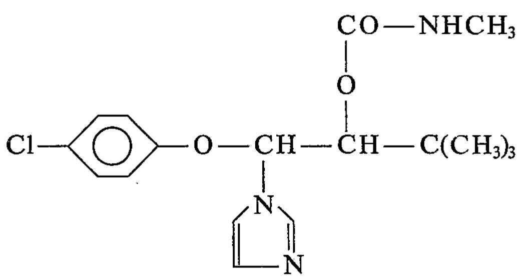

(E) 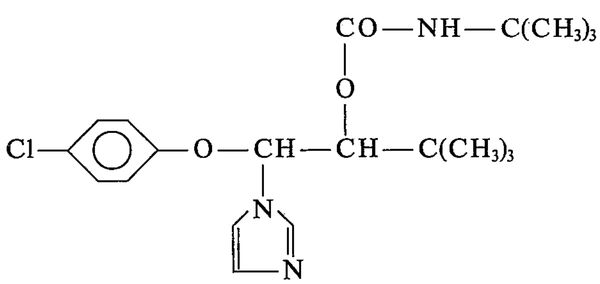

### Beispiel A

Sproßbehandlungs-Test/Getreidemehltau/Protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die

gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21–22°C und einer Luftfeuchtigkeit von 80–90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen eine sehr gute Wirkung, die derjenigen der aus dem Stand der Technik bekannten Verbindung (A) überlegen ist:

Verbindungen gemäß Herstellungsbeispiel 1.

## Beispiel B

### Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykolether auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80–90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

In diesem Test zeigen z. B. die folgenden Verbindungen eine sehr gute Wirkung, die derjenigen der aus dem Stand der Technik bekannten Verbindung (B) überlegen ist.

Verbindungen gemäß Herstellungsbeispiel 2.

## Beispiel C

### Erysiphe-Test (Gurken)/Protektiv

Lösungsmittel: 4,7 Gewichtsteile Aceton
Emulgator: 0,3 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe.

Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24°C und bei einer relativen Luftfeuchtigkeit von ca. 75% im Gewächshaus aufgestellt.

Nach 12 Stunden wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. folgende Verbindungen eine sehr gute Wirkung, die derjenigen der aus dem Stand der Technik bekannten Verbindungen (A) und (C) überlegen ist:

Verbindungen gemäß Herstellungsbeispielen 1 und 2.

Beispiel D

Podosphaera-Test (Apfel)/protektiv

Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von 70% gebraucht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindungen (C) und (D) überlegen ist:

Verbindungen gemäß Herstellungsbeispielen 1 und 2.

Beispiel E

Myzelwachstums-Test

Verwendeter Nährboden:

20 Gewichtsteile Agar-Agar
200 Gewichtsteile Kartoffeldekokt
5 Gewichtsteile Malz
15 Gewichtsteile Dextrose
5 Gewichtsteile Pepton
2 Gewichtsteile $Na_2HPO_4$
0,3 Gewichtsteile $Ca(NO_3)_2$

Verhältnis von Lösungsmittelgemisch zum Nährboden:

2 Gewichtsteile Lösungsmittelgemisch
100 Gewichtsteile Agarnährboden

Zusammensetzung Lösungsmittelgemisch:

0,19 Gewichtsteile DMF oder Aceton
0,01 Gewichtsteile Emulgator Alkylarylpolyglykolether
1,80 Gewichtsteile Wasser

2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach Wachstumsgeschwindigkeit der Pilze nach 4–10 Tagen. Bei der Auswertung wird das radiale Myzelwachstum auf den behandelten Nährboden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z. B. die folgenden Verbindungen eine sehr gute Wirkung, die derjenigen der aus dem Stand der Technik bekannten Verbindungen (D) und (E) überlegen ist:

Verbindungen gemäß Herstellungsbeispiel 2.

**Patentansprüche**

1. Acylierte Imidazolyl-γ-fluorpinakolyl-Derivate der allgemeinen Formel I

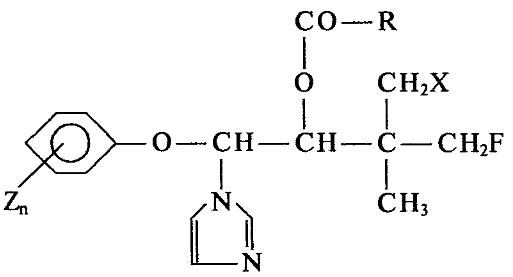

(I)

in welcher

R für Alkyl mit 1 bis 6 Kohlenstoffatomen, für 1-Methyl-vinyl, für Halogenalkyl mit 1 bis 3 Kohlenstoff- und 1 bis 5 Halogenatomen steht, für Alkoxy und Alkoxyalkyl mit 1 bis 4 Kohlenstoffatomen in jedem Alkylteil und für Cyclohexyl steht, weiterhin für Phenyl-, Phenylalkyl- und Phenoxyalkyl-Gruppen mit jeweils bis zu 2 Kohlenstoffatomen im Alkylteil steht, wobei die drei letztgenannten Gruppen im Phenylteil substituiert sein können durch Halogen, Alkyl und Alkoxy mit jeweils 1 bis 2 Kohlenstoffatomen, sodann für Alkylamino mit 1 bis 12, für Dialkylamino mit 1 bis 2 Kohlenstoffatomen in jedem Alkylteil und für Halogenalkylamino mit bis zu 4 Kohlenstoff- und bis zu 5 Halogenatomen steht, ferner für Alkoxycarbonylamino und Alkoxyalkylamino mit jeweils 1 bis 4 Kohlenstoffatomen in jedem Alkylteil steht, und schließlich noch für Phenylamino steht, welches durch Halogen substituiert sein kann,

X für Wasserstoff oder Fluor steht,

Z für Halogen, Cyano und Nitro, für Alkyl mit bis zu 4 Kohlenstoff- und für Halogenalkyl mit bis zu 2 Kohlenstoff- und bis zu 5 Halogenatomen und für Cyclohexyl steht, ferner für Alkoxycarbonyl mit insgesamt bis zu 5 und für Alkoxy und Alkylthio mit jeweils bis zu 2 Kohlenstoffatomen steht, und weiterhin für Phenyl, Phenoxy und Benzyl steht, wobei die drei letztgenannten Gruppen im Phenylteil durch Halogen substituiert sein können, und

n für ganze Zahlen von 0 bis 3 steht,

und deren physiologisch verträgliche Säureadditionssalze und Metallsalz-Komplexe.

2. Verfahren zur Herstellung von acylierten Imidazolyl-γ-fluorpinakolyl-Derivaten der allgemeinen Formel I, dadurch gekennzeichnet, daß man 1-Imidazolyl-2-hydroxybutan-Derivate der allgemeinen Formel II

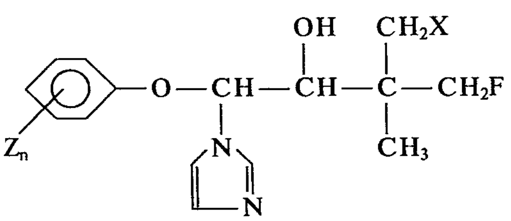

(II)

in welcher

X, Z und n die in Anspruch 1 angegebene Bedeutung haben,

(a) mit Säurehalogeniden der allgemeinen Formel III

Hal–CO–R (III)

in welcher

R die in Anspruch 1 angegebene Bedeutung hat und
Hal für Halogen steht,

gegebenenfalls in Gegenwart eines Lösungsmittels und gegebenenfalls in Gegenwart eines Säurebindemittels umsetzt, oder

(b) mit Säureanhydriden der allgemeinen Formel IV

R–CO–O–CO–R (IV)

in welcher

R die in Anspruch 1 angegebene Bedeutung hat,
in Gegenwart eines Lösungsmittels und gegebenenfalls in Gegenwart eines Katalysators umsetzt, oder

(c) mit Ketenen der allgemeinen Formel V

$$O{=}C{=}C(R'')-R' \qquad (V)$$

in welcher

R′ für Wasserstoff, für Alkyl mit 1 bis 5 Kohlenstoffatomen und für Halogenmethyl mit 1 bis 3 Halogenatomen steht, und weiterhin für Halogen, für die Alkoxygruppe mit 1 bis 3 Kohlenstoffatomen und für Phenyl steht, welches gegebenenfalls durch Halogen oder Alkyl mit 1 bis 2 Kohlenstoffatomen substituiert sein kann, und
R″ die unter R′ genannten Bedeutungen annehmen kann, jedoch mit der Maßgabe, daß die Summe der Kohlenstoffatome in den Alkylresten für R′ und R″ gemeinsam um die Zahl 1 unter der Zahl derjenigen Kohlenstoffatome, die im Anspruch 1 bei der R-Definition für die Alkylreste angegeben ist, bleiben muß, und daß höchstens einer der Reste R′ und R″ für gegebenenfalls wie angegeben substituiertes Phenyl, für die Alkoxygruppe oder Halogenmethyl stehen darf,

in Gegenwart eines Lösungsmittels und gegebenenfalls in Gegenwart eines Katalysators umsetzt, oder

(d) mit Isocyanaten der allgemeinen Formel VI

O=C=N–R‴ (VI)

in welcher

R‴ für Alkyl mit 1 bis 12 Kohlenstoffatomen, für Halogenalkyl mit 1 bis zu 4 Kohlenstoff- und bis zu 5 Halogenatomen steht, sowie für Alkoxycarbonyl und Alkoxyalkyl mit jeweils 1 bis 4 Kohlenstoffatomen in jedem Alkylteil und für Phenyl steht, welches gegebenenfalls durch Halogen substituiert sein kann,

in Gegenwart eines Lösungsmittels und gegebenenfalls in Gegenwart eines Katalysators umsetzt, und gegebenenfalls noch durch Umsetzen mit physiologisch verträglichen Säuren in die Säureadditions-Salze bzw. durch Reaktion mit Metallsalzen in die entsprechenden Metallsalzkomplexe überführt.

3. Fungizide Mittel, gekennzeichnet durch einen Gehalt an mindestens einem acylierten Imidazolyl-$\gamma$-fluorpinakolyl-Derivat der allgemeinen Formel I in Anspruch 1.

4. Verwendung von acylierten Imidazolyl-$\gamma$-fluorpinakolyl-Derivaten der allgemeinen Formel I in Anspruch 1 zur Bekämpfung von Pilzen, soweit sie nicht die Human- und Veterinärmedizin betrifft.

5. Verfahren zur Herstellung von fungiziden Mitteln, dadurch gekennzeichnet, daß man acylierte Imi-

dazolyl-γ-fluorpinakolyl-Derivate der allgemeinen Formel I in Anspruch 1 mit Streckmitteln und/oder oberflächenaktiven Mitteln vermischt.

## Claims

1. Acylated imidazolyl-γ-fluoropinacolyl derivatives of the general formula I

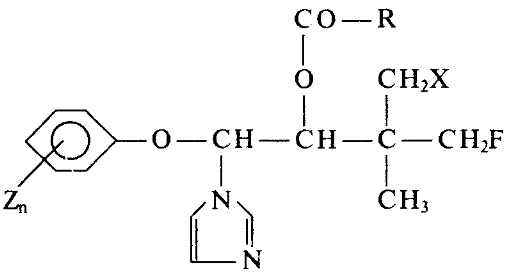

(I)

in which

R represents alkyl with 1 to 6 carbon atoms, 1-methyl-vinyl, halogenoalkyl with 1 to 3 carbon atoms and 1 to 5 halogen atoms, or represents alkoxy or alkoxyalkyl with 1 to 4 carbon atoms in each alkyl part or cyclohexyl, or furthermore represents phenyl, phenylalkyl or phenoxyalkyl groups with in each case up to 2 carbon atoms in the alkyl part, it being possible for the three last-mentioned groups to be substituted in the phenyl part by halogen, alkyl and alkoxy with in each case 1 to 2 carbon atoms, or then represents alkylamino with 1 to 12, dialkylamino with 1 to 2 carbon atoms in each alkyl part or halogenoalkylamino with up to 4 carbon atoms and up to 5 halogen atoms, or further represents alkoxycarbonylamino or alkoxyalkylamino with in each case 1 to 4 carbon atoms in each alkyl part, or finally also represents phenylamino which can be substituted by halogen,

X represents hydrogen or fluorine,

Z represents halogen, cyano or nitro, alkyl with up to 4 carbon atoms or halogenoalkyl with up to 2 carbon atoms and up to 5 halogen atoms or cyclohexyl, or further represents alkoxycarbonyl with a total of up to 5 or alkoxy or alkylthio with in each case up to 2 carbon atoms, or further represents phenyl, phenoxy or benzyl, it being possible for the three last-mentioned groups to be substituted in the phenyl part by halogen, and

n represents integers from 0 to 3,

and physiologically acceptable acid addition salts and metal salt complexes thereof.

2. Process for the preparation of acylated imidazolyl-γ-fluoropinacolyl derivatives of the general formula I, characterised in that 1-imidazolyl-2-hydroxy-butane derivatives of general formula II

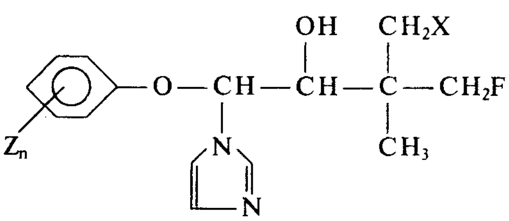

(II)

in which

X, Z and n have the meaning indicated in Claim 1,

(a) are reacted with acid halides of the general formula III

Hal–CO–R (III)

in which

R has the meaning indicated in Claim 1 and
Hal represents halogen,

if appropriate in the presence of a solvent and if appropriate in the presence of an acid-binding agent, or

(b) are reacted with acid anhydrides of the general formula IV

R–CO–O–CO–R (IV)

in which

R has the meaning indicated in Claim 1,
in the presence of a solvent and if appropriate in the presence of a catalyst, or

(c) are reacted with ketenes of the general formula V

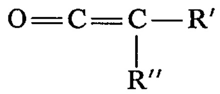 (V)

in which

R′ represents hydrogen, alkyl with 1 to 5 carbon atoms or halogenomethyl with 1 to 3 halogen atoms, or furthermore represents halogen, the alkoxy group with 1 to 3 carbon atoms or phenyl, which can optionally be substituted by halogen or alkyl with 1 to 2 carbon atoms, and
R″ can have the meanings mentioned under R′, however with the proviso that the sum of the carbon atoms in the alkyl radicals for R′ and R″ together must remain below the number of those carbon atoms which is indicated in Claim 1, in the R definition, for the alkyl radicals, by the number 1, and that not more than one of the radicals R′ and R″ may represent phenyl, which is optionally substituted as indicated, the alkoxy group or halogenomethyl,

in the presence of a solvent and if appropriate in the presence of a catalyst, or

(d) are reacted with isocyanates of the general formula VI

O=C=N–R‴ (VI)

in which

R‴ represents alkyl with 1 to 12 carbon atoms, halogenoalkyl with 1 up to 4 carbon atoms and up to 5 halogen atoms, or represents alkoxycarbonyl or alkoxyalkyl with in each case 1 to 4 carbon atoms in each alkyl part or represents phenyl which can optionally be substituted by halogen,

in the presence of a solvent and if appropriate in the presence of a catalyst, and, if required, the compounds obtained are then converted, by reaction with physiologically acceptable acids, into the acid addition salts, or, by reaction with metal salts, into the corresponding metal salt complexes.

3. Fungicidal compositions, characterised in that they contain at least one acylated imidazolyl-γ-fluoropinacolyl derivative of the general formula I in Claim 1.

4. Use of acylated imidazolyl-γ-fluoropinacolyl derivatives of the general formula I in Claim 1 for combating fungi, so far as it is not related to human and veterinary medicine.

5. Process for the preparation of fungicidal compositions, characterised in that acylated imidazolyl-γ-fluoropinacolyl derivatives of the general formula I in Claim 1 are mixed with extenders and/or surface-active agents.

**Revendications**

1. Dérivés d'imidazolyl-$\gamma$-fluoropinacolyle acylés de formule générale I

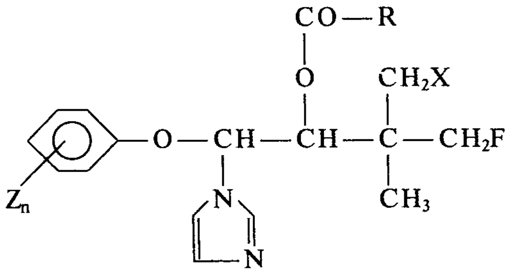

(I)

dans laquelle

R est un groupe alkyle ayant 1 à 6 atomes de carbone, un groupe 1-méthylvinyle, un groupe halogénalkyle ayant 1 à 3 atomes de carbone et 1 à 5 atomes d'halogènes, des groupes alkoxy et alkoxyalkyle ayant 1 à 4 atomes de carbone dans chaque partie alkyle et le groupe cyclohexyle, en outre, le groupe phényle, des groupes phénylalkyle ou phénoxyalkyle ayant dans chaque cas jusqu'à 2 atomes de carbone dans la partie alkyle, les trois groupes mentionnés en dernier lieu pouvant être substitués dans la partie phényle par un halogène, un radical alkyle et un radical alkoxy ayant chacun 1 ou 2 atomes de carbone, en outre, un groupe alkylamino ayant 1 à 12 atomes de carbone, un groupe dialkylamino ayant 1 ou 2 atomes de carbone dans chaque partie alkyle et un groupe halogénalkylamino ayant jusqu'à 4 atomes de carbone et jusqu'à 5 atomes d'halogènes, en outre, un groupe alkoxycarbonylamino et un groupe alkoxyalkylamino ayant chacun 1 à 4 atomes de carbone dans chaque partie alkyle, enfin le groupe phénylamino, qui peut être substitué par un halogène,

X est l'hydrogène ou le fluor,

Z est un halogène, le groupe cyano et le groupe nitro, un groupe alkyle ayant jusqu'à 4 atomes de carbone et un groupe halogénalkyle ayant jusqu'à 5 atomes d'halogènes et le groupe cyclohexyle, en outre, des groupes alkoxycarbonyle ayant au total jusqu'à 5 atomes de carbone et des groupes alkoxy et alkylthio ayant chacun jusqu'à 2 atomes de carbone, et en outre le groupe phényle, le groupe phénoxy et le groupe benzyle, les trois groupes mentionnés en dernier lieu pouvant être substitués par un halogène dans la partie phényle, et

n représente des nombres entiers de 0 à 3,

et leurs sels d'addition d'acides et leurs complexes de sels métalliques physiologiquement acceptables.

2. Procédé de production de dérivés d'imidazolyl-$\gamma$-fluoropinacolyle acylés de formule générale I, caractérisé en ce qu'on fait réagir des dérivés de 1-imidazolyl-2-hydroxybutane de formule générale II

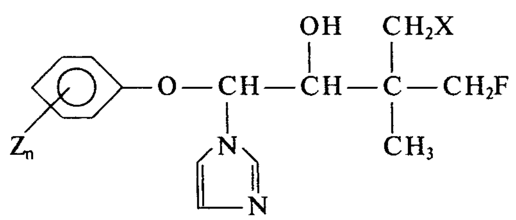

(II)

dans laquelle

X, Z et n ont la définition indiquée dans la revendication 1,

(a) avec des halogénures d'acides de formule générale III

Hal—CO—R (III)

dans laquelle

R a la définition indiquée dans la revendication 1 et
Hal représente un halogène,
éventuellement en présence d'un solvant et en la présence éventuelle d'un accepteur d'acide ou

(b) avec des anhydrides d'acides de formule générale IV

R—CO—O—CO—R (IV)

dans laquelle

R a la définition indiquée dans la revendication 1,
en présence d'un solvant et, le cas échéant en présence d'un catalyseur, ou
(c) avec des cétènes de formule générale V

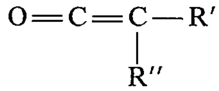 (V)

dans laquelle

R' désigne l'hydrogène, un groupe alkyle ayant 1 à 5 atomes de carbone et un groupe halogénométhyle ayant 1 à 3 atomes d'halogènes, en outre, un halogène, un groupe alkoxy ayant 1 à 3 atomes de carbone et le groupe phényle qui peut éventuellement être substitué par un halogène ou par un radical alkyle ayant 1 ou 2 atomes de carbone, et

R'' peut prendre les définitions mentionnées pour R', mais à condition que la somme des atomes de carbone des restes alkyle de R' et R'' reste inférieure d'une unité au nombre d'atomes de carbone qui est indiqué dans la revendication 1 pour les restes alkyle à propos de la définition de R, et qu'au maximum l'un des restes R' et R'' représente un groupe phényle éventuellement substitué comme indiqué, un groupe alkoxy ou un groupe halogénométhyle,

en présence d'un solvant et, le cas échéant en présence d'un catalyseur, ou
(d) avec des isocyanates de formule générale VI

O=C=N—R''' (VI)

dans laquelle

R''' est un groupe alkyle ayant 1 à 12 atomes de carbone, un groupe halogénalkyle ayant 1 à 4 atomes de carbone et jusqu'à 5 atomes d'halogènes, ainsi que des groupes alkoxycarbonyle et alkoxyalkyle ayant dans chaque cas 1 à 4 atomes de carbone dans chaque partie alkyle et le groupe phényle qui peut éventuellement être substitué par un halogène,

en présence d'un solvant et, le cas échéant, en présence d'un catalyseur, et on les transforme encore éventuellement par réaction avec des acides acceptables du point de vue physiologique en les sels d'addition d'acides ou, par réaction avec des sels métalliques, en les complexes de sels métalliques correspondants.

3. Compositions fongicides, caractérisées par une teneur en au moins un dérivé d'imidazolyl-$\gamma$-fluoropinacolyle acylé de formule générale I suivant la revendication 1.

4. Utilisation de dérivés d'imidazolyl-$\gamma$-fluoropinacolyle acylés de formule générale I suivant la revendication 1 pour la lutte contre des champignons, dans la mesure où elle ne concerne pas la médecine humaine et la médecine vétérinaire.

5. Procédé de production de compositions fongicides, caractérisé en ce qu'on mélange des dérivés d'imidazolyl-$\gamma$-fluoropinacolyle acylés de formule générale I suivant la revendication 1 avec des diluants et/ou avec des agents tensio-actifs.